# EUROPEAN PATENT SPECIFICATION

(11) **EP 2 629 600 B1**
(45) Date of publication and mention of the grant of the patent: **15.09.2021**
(21) Application number: 10858733.8
(22) Date of filing: 19.10.2010
(51) Int. Cl.: A01K 1/015, C04B 11/00

(54) **METHOD FOR PREPARING A HARDENED CALCIUM SULFATE DIHYDRATE BLOCK**
VERFAHREN ZUR HERSTELLUNG EINES GEHÄRTETEN CALCIUMSULFAT-DIHYDRAT-BLOCKS
PROCÉDÉ PERMETTANT DE PRÉPARER UN BLOC DE SULFATE DE CALCIUM BIHYDRATÉ DURCI

(43) Date of publication of application: 28.08.2013
(73) Proprietor: Lin, Jiin-Huey Chern, Winnetka, MO 60093 (US); Ju, Chien-Ping, Kansas City, MO 64137 (US)
(72) Inventor: Lin, Jiin-Huey Chern, Winnetka, MO 60093 (US); Ju, Chien-Ping, Kansas City, MO 64137 (US)
(74) Representative: Grünecker Patent- und Rechtsanwälte PartG mbB
(86) International application number: PCT/US2010/002776
(87) International publication number: WO 2012/054009

(56) References cited:
- EP-A1- 1 208 850
- US-A- 5 462 722
- US-A1- 2008 050 407
- US-A1- 2009 192 629

## Description

### Field of the Invention

The present invention is related to a method for preparing a hardened calcium sulfate dihydrate block from a paste of calcium sulfate hemihydrate powder and an aqueous solution.

### Background of the Invention

For many orthopedic applications, calcium sulfate dihydrate is not an ideal implant material due to its insufficient compressive strength and/or a dissolution rate that is too high in comparison with the calcium phosphate implant material. Furthermore, one major drawback of calcium sulfate hemihydrate bone cement is that it conventionally has a working time and setting time which are not sufficiently long for operation. Accordingly, researchers have been trying various approaches to (but still are not successful to) develop calcium sulfate hemihydrate bone cement with suitable working time and setting time and with a desired compressive strength of the resultant hardened calcium sulfate dihydrate block.

US 5462722A discloses inorganic composite materials for hard tissue replacement.

US 2009/0192629 A1 discloses an injectable composition for a bone mineral substitute material, which comprises a dry powder mixed with an aqueous liquid.

EP 1 208 850 A1 discloses an osteogenesis promoter sustained-release paste.

### Summary of the Invention

A primary object of the present invention is to provide a method for preparing a hardened calcium sulfate dihydrate block.

The present invention provides a method for preparing a hardened calcium sulfate dihydrate block comprising mixing calcium sulfate hemihydrate powder, preferably alpha-type calcium sulfate hemihydrate powder, with an aqueous solution containing phosphate ions so as to form a paste, wherein the aqueous solution has a pH value lower than 10, and forming a block of hardened calcium sulfate dihydrate from said paste, wherein said aqueous solution is an aqueous solution of (NH₄)₃PO₄, (NH₄)₂HPO₄, NH₄H₂PO₄, K₃PO₄, K₂HPO₄, Na₃PO₄, NaH₂PO₄, H₃PO₄, or a mixture thereof.

Preferred embodiments are set forth in the subclaims.

### Detailed Description of the Invention

The present invention provides a technique for prolonging the working time and setting time of a calcium sulfate hemihydrate paste by mixing calcium sulfate hemihydrate powder with an aqueous solution containing phosphate ions, so that the paste is suitable for operation and the resultant hardened calcium sulfate dihydrate block from the paste also has an improved mechanical strength. A bone cement formula provided according to the present invention is composed of calcium sulfate hemihydrate powder, an aqueous solution containing phosphate ions, and one or more optionally functional components such as living cells, a growth factor, a drug or a pore-forming agent.

The present invention will be better understood through the following examples which are illustrative only.

### Examples:

Chemicals used in the examples:

| Chemical | Formula | Supplier |
|---|---|---|
| calcium sulfate hemihydrate (CSH) | CaSO₄·0.5H₂O | Showa, Tokyo, Japan |
| calcium sulfate Dihydrate (CSH) | CaSO₄·2H₂O | Panreac, E.U. |
| Calcium sulfate | CaSO₄ | Showa, Tokyo, Japan |
| Diammonium hydrogen phosphate | (NH₄)₂HPO₄ | Showa, Tokyo, Japan |
| Disodium hydrogen phosphate | Na₂HPO₄ | Showa, Tokyo, Japan |
| phosphoric acid | H₃PO₄ | Showa, Tokyo, Japan |
| hydrochloric acid | HCl | Nihon Shiyaka, Osaka, Japan |
| sodium hydroxide | NaOH | Showa, Tokyo, Japan |
| Dipotassium hydrogen phosphate | K₂HPO₄ | Katayama, Osaka, Japan |
| Diammonium dihydrogen phosphate | (NH₄) H₂PO₄ | Showa, Tokyo, Japan |
| Sodium dihydrogen phosphate | NaH₂PO₄ | Showa, Tokyo, Japan |
| Potassium dihydrogen phosphate | KH₂PO₄ | Icatayama, Tokyo, Japan |
| Tartaric acid | C₂H₂(OH)₂(COOH)₂ | Katayam, Osaka, Japan |
| Malic acid | C₂H₃(OH)(COOH)₂ | Pantreac, Barcelona, Spain |
| Potassium chloride | KCl | Showa, Tokyo, Japan |
| Sodium chloride | NaCl | Katayam, Osaka, Japan |
| Potassium dihydrogen phosphate | KH₂PO₄ | Showa, Tokyo, Japan |
| Sodium hydrogen carbonate | NaHCO₃ | Atayam, Osaka, Japan |

### Preparation of a calcium sulfate cement paste

The calcium sulfate cement paste was prepared by mixing appropriate amounts of CSH powder and hardening solution (for example, diammonium dihydrogen phosphate or dipotassium hydrogen phosphate) with a desirable liquid/powder ratio (for example, 0.35 cc/g).

### Preparation of a calcium sulfate dense block article

Appropriate amounts of CSH powder and hardening solution were uniformly mixed in a ball miller at a desirable L/P ratio to form a calcium sulfate cement paste.

Prior to being fully hardened, the paste was placed in a mold under a desirable pressure (for example, 4413 N (450 Kgf)) to squeeze a portion of the hardening solution out of the paste to form a hardened dense block. After being removed from the mold, one group of the hardened samples was placed in a moisture-proof container for 1 day. Another group of samples was further impregnated in an impregnation solution (for example, (NH₄)₂HPO₄ or K₂HPO₄) at a desirable temperature (for example, 37°C) for a period of time (for example, 1 day), followed by drying in an oven at 50°C for 1 day.

### Preparation of a calcium sulfate porous block article

Appropriate amounts of CSH powder, pore-forming particles (for example KCl) and hardening solution were uniformly mixed with a desirable KCl/CSH ratio (for example 1:1 or 1.5:1 by weight) and a desirable liquid/powder ratio (for example, 0.35 cc/g) to form a KCl/CSH cement paste.

Prior to being fully hardened, the KCl/CSH cement paste was placed in a mold under a desirable pressure (for example, 4413 N (450Kgf)) to squeeze a portion of the hardening solution out of the paste to for a hardened dense block. After being removed from the mold, the hardened dense block sample was immersed in de-ionized water for a period of time (for example, 3 days) to allow the pore-forming particles to be washed out of the dense block to form a calcium sulfate porous block, followed by drying in an oven at 50°C for 1 day.

One group of the porous block samples was further impregnated in an impregnation solution (e.g. (NH₄)₂HPO₄ or K₂HPO₄) at a desirable temperature (for example 37°C or 4°C) for a period of time to allow the strength of the porous block to increase, followed by drying in an oven at 50°C for 1 day. To remove the residual impregnation solution from inside the pores, the impregnated porous samples were rinsed in de-ionized water for a period of time (for example, 3 days).

Another group of samples was further treated by immersion in CaCl₂ solution for a period of time (for example, 3 days) to further enhance the strength of the porous samples.

### Compressive strength (CS) testing

To measure the CS of a hardened cement, after mixing for 1 min, the cement paste was packed in a 6 mm diameter, 12 mm deep cylindrical stainless steel mold under a pressure of 0.7 MPa for 30 min. After being removed from the mold, the hardened cement samples were immersed in Hanks' physiological solution which was maintained at 37°C and agitated daily to help maintain uniform ion concentrations. After immersion, samples were removed from the solution for CS testing while samples are still wet. The CS testing was conducted using a desk-top mechanical tester (Shimadzu AGS-500D, Tokyo, Japan) at a crosshead speed of 1.0 mm/min. The test method is according to ASTM 451-99a method.

### Working time (WT)/setting time (ST) measurement

The working time of cement paste was determined by the time after that the cement paste was no longer workable. The setting time of cement paste was measured according to the standard method set forth in ISO 1566 for dental zinc phosphate cements. The cement is considered set when a 400 gm weight loaded onto a Vical needle with a 1 mm diameter tip fails to make a perceptible circular indentation on the surface of the cement.

### pH measurement

The early stage (during setting process) variation in pH was determined using a pH meter (Suntex Instruments SP20004, Taipei, Taiwan) that was buried in the cement paste immediately after the powder and setting liquid were mixed. The first reading was taken at 1 minute after mixing. The measurement was continued until the paste nearly becomes set. Readings were taken every 30 seconds until 30 minutes after mixing. After then they were taken every 60 seconds.

The variation in pH value of Hanks' solution in which the cement paste sample was immersed was monitored using the same pH meter. 2 g cement paste was taken after mixing the powder and the setting solution for 5 minutes, and it was immersed in 20 ml Hanks' solution with a pH value of 7.05 for the test. The solution was maintained at 37°C throughout testing and continually stirred to help maintain uniform ion concentrations of the solution.

Composition of Hanks' solution (Hench, 1971)

| Component | Concentration (g/L) |
|---|---|
| NaCl | 8.00 |
| Na₂HPO₄·2H₂O | 0.06 |
| CaCl₂ | 0.14 |
| NaHCO₃ | 0.35 |
| KCl | 0.40 |
| Glucose | 1.00 |
| MgCl₂·6H₂O | 0.10 |
| MgSO₄·7H₂O | 0.06 |
| KH₂PO₄ | 0.06 |

### Measurement of porosity

The porosity of the various samples was measured according to ASTM C830-00 (2006) method, "Standard Test Methods for Apparent Porosity, Liquid Absorption, Apparent Specific Gravity, and Bulk Density of Refractory Shapes by Vacuum Pressure".

### RESULTS

### GROUP A-PASTE

**Table A-1. Working time, setting time and CS of calcium sulfate cement prepared from acidic hardening solution (L/P=0.35) (n=6) (a hardening solution of KH₂PO₄ is not according to the invention)**

| Hardening solution | Conc. (M) | Solution pH | WT (Min) | ST (Min) | CS (MPa) |
|---|---|---|---|---|---|
| Water | | 7.0 | 2.0 | 2.4 | 12.7±2.9 |
| H₃PO₄ | 0.01875 | 2.2 | 5.3 | 6:7 | 29.1±1.2 |
| | 0.0375 | 2.1 | 8.5 | 10.3 | 33.7±4.3 |
| | 0.075 | 1.9 | 8.3 | 10.8 | 30.3±2.0 |
| KCl | 0.01875 | 6.7 | 1.3 | 1.5 | 11.5±2.4 |
| | 0.0375 | 6.7 | 1.7 | 1.3 | 6.5±1.7 |
| | 0.075 | 6.9 | 1.8 | 1.4 | 3.8±1.9 |
| HCl | 0.01875 | 2.1 | 2.5 | 2.8 | 16.2±1.6 |
| | 0.0375 | 2.0 | 3.2 | 3.5 | 19.4±1.7 |
| | 0.075 | 1.5 | 3.8 | 4.2 | 22.2±2.4 |
| HNO₃ | 0.01875 | 1.8 | 1.9 | 2.2 | 17.7±2.8 |
| | 0.0375 | 1.5 | 2.5 | 2.8 | 15.0±3.4 |
| | 0.075 | 1.3 | 2.2 | 2:7 | 17.1±1.5 |
| KH₂PO₄ | 0.01875 | 5.1 | 4.6 | 4.9 | 25.5±1.8 |
| | 0.0375 | 4.8 | 5.7 | 6.3 | 23.3±2.9 |
| | 0.075 | 4.6 | 13.4 | 13.8 | 18.7±3.7 |

**Table A-2. Working time, setting time and CS of calcium sulfate cement prepared from basic hardening solution (L/P=0.35) (n=6) (a hardening solution of Na₂HPO₄ is not according to the invention)**

| Hardening solution | Conc. (M) | Solution pH | WT (Min) | ST (Min) | CS (MPa) |
|---|---|---|---|---|---|
| Water | | 7.0 | 2.0 | 2.4 | 12.7±2.9 |
| (NH₄)₂HPO₄ | 0.01875 | 7.8 | 5.5 | 6.3 | 27.5±2.5 |
| | 0.0375 | 7.9 | 8.3 | 10.3 | 33.6±3.6 |
| | 0.075 | 8.0 | 10.2 | 10.8 | 32.0±1.8 |
| K₂HPO₄ | 0.01875 | 8.4 | 4.7 | 5.0 | 28.2±2.9 |
| | 0.0375 | 8.4 | 6.3 | 6.6 | 36.4±2.2 |
| | 0.075 | 9.2 | 5.8 | 6.3 | 33.9±4.0 |
| NaHCO₃ | 0.01875 | 8.5 | 2.9 | 3.5 | 11.6±1.9 |
| | 0.0375 | 8.6 | 3.5 | 4.1 | 11.6±2.2 |
| | 0.075 | 8.6 | 4.3 | 4.8 | 14.2±3.5 |
| NaOH | 0.01875 | 12.1 | 2.4 | 2.5 | 10.1±1.2 |
| | 0.0375 | 12.4 | 2.1 | 2.2 | 9.8±3.4 |
| | 0.075 | 12.7 | 2.0 | 2.1 | 8.8±2.9 |
| NaCl | 0.01875 | 7.2 | 2.0 | 2.5 | 8.6±1.9 |
| | 0.0375 | 7.0 | 1.7 | 2.4 | 7.2±2.7 |
| | 0.075 | 7.5 | 1.7 | 2.3 | 7.0±1.7 |
| Na₂HPO₄ | 0.01875 | 8.4 | 5.8 | 6.3 | 30.9±0.8 |
| | 0.0375 | 8.8 | 5.2 | 6.2 | 34.0±1.3 |
| | 0.075 | 9.4 | 8.3 | 9.2 | 27.2±2.9 |
| Na₂CO₃ | 0.01875 | 10.7 | 2.3 | 2.8 | 18.4±1.3 |
| | 0.0375 | 10.8 | 3.5 | 4.0 | 19.0±2.4 |
| | 0.075 | 11.0 | 2.2 | 2.5 | 16.4±2.0 |

### Summary (Tables A-1 and A-2):

1. All solutions with phosphate (no matter acidic or basic) lead to reasonable WT/ST (about 5-10 min), while all solutions without phosphate (no matter acidic or basic) result in too short WT/ST (for paste injection surgery).
   (Note: Too short WT/ST leave insufficient time for preparation and/or surgery)
2. All solutions with phosphate (no matter acidic or basic) give much higher CS values than those without phosphate.
3. Phosphate concentration of 0.0375 M leads to highest CS value, although CS values from all three concentrations (0.01875-0.075 M) are acceptable.

**Table A-3. Effect of concentration of (NH₄)₂HPO₄ hardening solution on WT and ST of calcium sulfate cement**

| (NH₄)₂HPO₄ conc. (M) | L/P ratio (cc/g) | WT (Min) | ST (Min) |
|---|---|---|---|
| 0.01875 | 0.35 | 5.5 | 6.3 |
| 0.0375 | 0.35 | 8.3 | 10.3 |
| 0.075 | 0.35 | 10.2 | 10.8 |
| 0.25 | 0.35 | 6.4 | 6.7 |
| 0.50 | 0.35 | 6.7 | 7.0 |
| 0.75 | 0.35 | 7.7 | 8.0 |
| 1.00 | 0.40* | 8.5 | 9.0 |

| | | | |
|---|---|---|---|
| *L/P ratio is increased to facilitate mixing | | | |

**Table A-4. Effect of concentration of K₂HPO₄ hardening solution on WT and ST of calcium sulfate cement**

| K₂HPO₄ conc. (M) | L/P ratio (cc/g) | WT (Min) | ST (Min) |
|---|---|---|---|
| 0.01875 | 0.35 | 4.7 | 5.0 |
| 0.0375 | 0.35 | 6.3 | 6.6 |
| 0.075 | 0.35 | 5.8 | 6.3 |
| 0.10 | 0.40* | 8.5 | 9.2 |
| 0.25 | 0.40* | 17.5 | 42.0 |

| | | | |
|---|---|---|---|
| * L/P ratio is increased to facilitate mixing | | | |

### Summary (Tables A-3 and A-4):

1. (NH₄)₂HPO₄ solution with phosphate concentration up to 1.0 M gives reasonable WT/ST.
2. K₂HPO₄ solution with phosphate concentration up to 0.1 M gives reasonable WT/ST. When K₂HPO₄ concentration is higher than 0.1 M, WT/ST becomes too long (for paste injection surgery).
   (Note: Too long WT/ST indicates a low early strength and easy dispersion of the paste upon contact body fluid/blood prior to hardening)

**Table A-5. Effect of concentration of (NH₄)₂HPO₄ hardening solution on CS of calcium sulfate cement (L/P=0.35) (n=6)**

| Hardening solution | Concentration (M) | CS (MPa) |
|---|---|---|
| Water | | 12.7±2.9 |
| (NH₄)₂HPO₄ | 0.01875 | 27.5±2.5 |
| | 0.0375 | 33.6±3.6 |
| | 0.075 | 32.0±1.8 |
| | 0.10 | 26.8±2.8 |
| | 0.25 | 20.7±4.1 |
| | 0.50 | 17.7±19.0 |
| | 0.75 | 13.3±13.7 |
| | 1.00* | 1.4±1.4 |
| | 2.00* | 3.6±3.7 |

| | | |
|---|---|---|
| *L/P ratio is increased to 0.40 to facilitate mixing. | | |

### Summary:

1. Phosphate concentration of 0.0375 M leads to highest CS value, although phosphate concentration up to 0.1 M still gives reasonable CS.
2. When phosphate concentration is 0.75 M, the CS value becomes lower than half that of 0.0375 M.

**Table A-6. Effect of pH value on WT and ST of calcium sulfate cement (n=6)**

| Hardening solution | pH value | L/P (cc/g) | WT (Min) | ST (Min) |
|---|---|---|---|---|
| Water | 7.0 | 0.40 | 2.0 | 2.4 |
| (NH₄)₂HPO₄ | 1.0 | 0.35 | 5.3 | 6.7 |
| | 3.0 | 0.35 | 7.8 | 8.2 |
| | 5.0 | 0.35 | 9.8 | 10.3 |
| | 7.9 | 0.35 | 8.3 | 10.3 |
| | 9.0 | 0.35 | 10.3 | 11.0 |
| | 11.0 | 0.40 | 3.3 | 3.7 |
| | 13.0 | 0.40 | 1.7 | 2.2 |
| K₂HPO₄ | 8.4 | 0.35 | 6.3 | 6.6 |
| | 9.0 | 0.35 | 5.8 | 6.3 |
| | 11.0 | 0.35 | 5.5 | 5.8 |
| | 13.0 | 0.40 | 2.3 | 2.5 |

### Summary:

1. WT/ST values are very sensitive to the pH value of the hardening solution.
2. When phosphate-containing solution (no matter (NH₄)₂HPO₄) or K₂HPO₄) has a pH value higher than about 11, WT/ST largely decreases to become unacceptably short.

**Table A-7. Effect of pH value on CS of calcium sulfate cement [Note: (NH₄)₂HPO₄ conc: 0.0375 M, pH=7.9; K₂HPO₄ conc. 0.0375 M, pH=8.4. Adding HCl to decrease pH value, while adding NaOH to increase pH value) (n=6)**

| Hardening solution | pH value | L/P (cc/g) | CS (MPa) |
|---|---|---|---|
| Water | 7.0 | 0.40 | 12.7±2.9 |
| (NH₄)₂HPO₄ | 1.0 | 0.35 | 30.4±2.0 |
| | 3.0 | 0.35 | 31.2±3.4 |
| | 5.0 | 0.35 | 29.9±3.1 |
| | 7.9 | 0.35 | 33.6±3.6 |
| | 9.0 | 0.35 | 32.1±2.1 |
| | 11.0 | 0.40 | 22.9±4.0 |
| | 13.0 | 0.40 | 10.8±1.6 |
| K₂HPO₄ | 1.0 | 0.35 | 19.5±1.0 |
| | 3.0 | 0.35 | 23.7±2.4 |
| | 5.0 | 0.35 | 24.9±0.8 |
| | 8.4 | 0.35 | 36.4±2.2 |
| | 11.0 | 0.35 | 36.2±1.3 |
| | 12.0 | 0.35 | 30.0±4.6 |
| | 13.0 | 0.40 | 11.9±1.4 |

### Summary:

1. CS values are very sensitive to the pH value of the hardening solution.
2. When (NH₄)₂HPO₄ hardening solution has a pH value higher than about 11, the CS value largely decreases. When its pH value reaches 13.0, the CS value decreases by 68% from its highest CS.
3. When K₂HPO₄ has a pH value higher than about 12.0, the CS value largely decreases. When its pH value reaches 13.0, the CS value decreases by 67% from its highest CS.

**Table A-8. Early stage pH variation of calcium sulfate cement paste prepared from 0.0375 M (NH₄)₂HPO₄ hardening solution**

| | | | | | | |
|---|---|---|---|---|---|---|
| Time (min) | 2.5 | 5.0 | 7.5 | 10.0 | 12.5 | 15.0 |
| Paste pH | 6.2 | 6.4 | 6.6 | 6.7 | 6.7 | 6.8 |

### Summary:

The pH value of the paste (mixing CSH and 0.0375M (NH₄)₂HPO₄) slowly increases to a range between 6.5 and 7.0 after mixing for 15 min, a range acceptable to tissues/cells.

**Table A-9. pH variation of Hanks' solution wherein calcium sulfate cement prepared from 0.0375 M (NH₄)₂HPO₄ hardening solution is immersed.**

| | | | | | |
|---|---|---|---|---|---|
| Immersion time (day) | 1 | 3 | 7 | 14 | 40 |
| Hanks' solution pH | 6.2±0.5 | 6.0±0.2 | 6.3±0.1 | 6.6±0.1 | 6.6±0.3 |

### Summary:

The pH value of the Hanks' solution is always between 6 and 7, a range acceptable to tissues/cells. After 14 days, it becomes close to neutral.

**Table A-10. CS variation of calcium sulfate cement prepared from de-ionized water, 0.0375 M (NH₄)₂HPO₄ and 0.0375 M K₂HPO₄ hardening solutions followed by immersion in Hanks' solution for different periods of time (n=6).**

| Hardening solution | Conc. (M) | Time (day) | CS (MPa) |
|---|---|---|---|
| De-ionized water | | 1 | 12.7±2.9 |
| | | 3 | 9.4±3.0 |
| | | 7 | 11.1±2.2 |
| | | 14 | 9.1±3.8 |
| | | 30 | 6.0±2.0 |
| (NH₄)₂HPO₄ | 0.0375 | 1 | 33.6±3.6 |
| | | 3 | 29.6±1.2 |
| | | 7 | 30.0±2.1 |
| | | 14 | 25.1±4.1 |
| | | 30 | 26.4±3.2 |
| K₂HPO₄ | 0.0375 | 1 | 36.4±2.2 |
| | | 3 | 24.1±1.8 |
| | | 7 | 18.8±4.2 |
| | | 14 | 19.0±2.0 |
| | | 30 | 16.9±1.6 |

### Summary:

1. The CS values of calcium sulfate cement prepared from de-ionized water are far lower than from other two phosphate-containing hardening solutions.
2. The CS values of calcium sulfate cement prepared from phosphate-containing hardening solutions decay reasonably slowly when stored in Hanks' solution for a long period of time.
3. The calcium sulfate cement prepared from (NH₄)₂HPO₄ hardening solution behaves exceptionally well in terms of maintaining CS value when stored in Hanks' solution. After 30 days, its CS value decreases only by less than 20%, compared to 1-day value.

**Table A-11. Effect of tartaric acid addition on CS of calcium sulfate cement prepared from 0.0375 M (NH₄)₂HPO₄ hardening solution**

| Hardening solution | 0.0375 M (NH₄)₂HPO₄ | 0.0375 M (NH₄)₂HPO₄/ tartaric acid (1:1 by wt) |
|---|---|---|
| CS (MPa) | 26.5 | 31.3 |

### Summary:

1. The CS value of calcium sulfate cement prepared from (NH₄)₂HPO₄/tartaric acid hardening solution is higher than that prepared from (NH₄)₂HPO₄ by 18%.

### GROUP B- DENSE BLOCK

[NOTE: For pre-formed block samples, "WT/ST" is no longer an issue and K₂HPO₄ was selected as hardening solution for study]

**Table B-1. Effect of K₂HPO₄ hardening solution concentration on CS of calcium sulfate dense block article (L/P=0.35)**

| K₂HPO₄ conc. (M) | CS (MPa) |
|---|---|
| 0.01875 | 97.1±2.6 |
| 0.0375 | 100.2±5.9 |
| 0.056 | 108.2±7.8 |
| 0.075 | 113.5±8.7 |
| 0.10 | 106.3±4.1 |
| 0.15 | 101.6±4.8 |
| 0.20 | 63.6±7.1 |
| 0.40 | 21.7±1.7 |
| 1.00 | 23.8±1.3 |

### Summary:

1. Calcium sulfate dense block samples prepared from K₂HPO₄ hardening solution with phosphate concentrations from 0.01875 M to 0.15 M have high CS values. When the concentration is higher than 0.20 M, CS largely decreases to unacceptably low values.
2. Concentration of 0.075 M results in the highest CS value.

**Table B-2. CS values of calcium sulfate dense block samples prepared from 0.075M K₂HPO₄ hardening solution (L/P=0.35) followed by impregnating treatment in different Impregnating solutions for 1 day. (n=6)**

| Impregnating solution | Impregnating temperature (°C) | CS (MPa) |
|---|---|---|
| None | | 113.5±8.7 |
| Hanks' solution | 0 | 111.9±4.2 |
| | 37 | 100.4±3.0 |
| K₂HPO₄ | 0 | 129.4±8.3 |
| 1M | 37 | 117.0±11.6 |
| NaOH | 0 | 81.4±7.4 |
| 1M | 37 | 78.7±6.8 |
| KCl | 0 | 74.6±9.5 |
| 3M | 37 | 74.0±7.0 |
| HNO₃ | 0 | 3.2±1.2 |
| 15.4M | 37 | 6.0±0.5 |

### Summary:

1. Dense block samples prepared from 0.075M K₂HPO₄ hardening solutions have much higher CS values after impregnating for 1 day in phosphate-containing solutions than in solutions without phosphate. [Note: HANKS' solution is a phosphate-containing solution]
2. Lower impregnation temperature (0°C) results in higher CS than higher impregnation temperature (37°C), no matter the impregnating solution is Hanks' solution or K₂HPO₄ solution.

**Table B-3. CS variation of calcium sulfate dense block prepared from 0.075M K₂HPO₄ hardening solution (L/P=0.35) followed by immersion in Hanks' solution for different periods of time.(n=6)**

| Hardening solution | Conc. (M) | Immersion time (day) | CS (MPa) |
|---|---|---|---|
| K₂HPO₄ | 0.0375 | None | 92.1±1.1 |
| | | 1 | 88.6±3.7 |
| | | 4 | 77.7±3.1 |
| | | 8 | 76.0±3.0 |
| | | 16 | 70.5±1.3 |
| | 0.075 | None | 113.5±8.7 |
| | | 1 | 99.7±3.0 |
| | | 4 | 87.3±1.2 |
| | | 8 | 86.2±3.0 |
| | | 16 | 78.7±2.7 |

### Summary:

1. Dense block samples prepared from K₂HPO₄ hardening solution (no matter the concentration is 0.0375 M or 0.075M) decay reasonably slowly in CS when immersed in Hanks' solution. After immersion for 16 days, they still maintain about 70% of their original CS values.

### GROUP C- POROUS BLOCK

### Preparation of calcium sulfate porous block samples

The calcium sulfate porous block samples for the study were prepared by first mixing CSH and KCl powders (1:1 by weight) with 0.075 M K₂HPO₄ hardening solution at a L/P ratio of 0.35 cc/g) to form a KCl/CSH cement paste. Prior to being fully hardened, the KCl/CSH cement paste was placed in a mold under a pressure of 4413 N (450Kgf) to squeeze a portion of the hardening solution out of the paste to form a hardened dense block. After being removed from the mold, the hardened dense block sample was immersed in de-ionized water for 3 days to allow the pore-forming particles to be washed out of the dense block to form a calcium sulfate porous block, followed by drying in an oven at 50°C for 1 day.. The X-ray diffraction results indicated that the KCl phase was totally dissolved (KCl peaks disappeared from the XRD patterns) after immersion in de-ionized water at 37C or 4C for 3 days.

One group of the porous block samples was further impregnated in an impregnation solution ((NH₄)₂HPO₄ or K₂HPO₄) at 37°C or 4°C for a period of time to allow the strength of the porous block to increase, followed by drying in an oven at 50°C for 1 day.

Another group of samples was further treated by immersion in CaCl₂ solution for 1-3 days to further enhance the strength of the porous samples.

**Table C-1. CS values of calcium sulfate porous block samples prepared by immersion for 3 days in de-ionized water at 37°C and 4°C to remove KCl particles.**

| | | |
|---|---|---|
| Immersion temperature (°C) | 37 | 4 |
| CS (MPa) | 2.8 | 4.8 |

### Summary:

1. The CS value of the porous block sample immersed in 4°C de-ionized water (4.8 MPa) is higher than that immersed in 37°C de-ionized water(2.8 MPa) by 71%.

**Table C-2. CS values of impregnation-treated porous block samples under different impregnating conditions. (The KCl particles were dissolved by immersion in 4°C de-ionized water for 3 days)**

| Impregnating solution (4°C) | Solution conc. (M) | Impregnating time (day) | CS (MPa) |
|---|---|---|---|
| None | - | - | 4.8±0.7 |
| (NH₄)₂HPO₄ | 1.0 | 1 | 4.7±0.8 |
| (NH₄)₂HPO₄ | 1.5 | 1 | 5.5±1.2 |
| (NH₄)₂HPO₄ | 2.0 | 1 | 7.0±1.8 |
| K₂HPO₄ | 1.0 | 1 | 4.5±1.1 |
| K₂HPO₄ | 1.0 | 2 | 5.0±1.4 |
| K₂HPO₄ | 1.0 | 3 | 4.3±1.2 |
| K₂HPO₄ | 1.5 | 1 | 5.4±1.1 |
| K₂HPO₄ | 1.5 | 2 | 5.4±0.9 |
| K₂HPO₄ | 1.5 | 3 | 5.9±1.1 |
| K₂HPO₄ | 2.0 | 1 | 6.2±1.0 |
| K₂HPO₄ | 2.0 | 3 | 10.2±2.0 |

### Summary:

1. In both impregnating solutions, CS values largely increase when the concentration of the impregnating solution increases.
2. After impregnating for 1 day, the CS increases by 46% in 2M (NH₄)₂HPO₄ and 29% in 2M K₂HPO₄ impregnating solution. After impregnating for 3 days, the CS increases by as much as 113% in 2M K₂HPO₄ impregnating solution.

### CaCl₂ treatment to further enhance CS

To further enhance the strength of the porous block sample, a calcium sulfate porous block sample with a CS value of 5.0 MPa (control) was immersed in CaCl₂ solution under different conditions

**Table C-3. CS values of calcium sulfate porous block samples treated by immersion in CaCl₂ solution under different conditions.**

| CaCl₂ solution conc. (M) | Immersion time (day) | CS (MPa) |
|---|---|---|
| Control | - | 5.0±0.2 |
| 1.0 | 1 | 13.7±1.7 |
| | 3 | 12.2±1.3 |
| | 5 | 9.6±0.7 |
| | 7 | 8.0±0.5 |
| 2.0 | 1 | 14.9±1.6 |
| | 3 | 21.1±2.5 |
| | 5 | 13.7±1.0 |
| | 7 | 11.0±1.0 |
| 3.0 | 1 | 17.3±1.9 |
| | 3 | 25.1±2.6 |
| | 5 | 17.0±2.3 |
| | 7 | 17.4±1.0 |
| 4.0 | 1 | 14.0±1.9 |
| | 3 | 13.3±2.9 |
| | 5 | 14.8±2.0 |
| | 7 | 17.3±3.6 |

### Results:

1. After CaCl₂ treatment, the CS largely increases under all conditions.
2. The largest increases in CS were found in the treatments of 2M CaCl₂ for 3 days (from 5.0 MPa to 21.1 MPa, an increase of 322%) and 3M CaCl₂ for 3 days (from 5.0 MPa to 25.1 MPa, an increase of 402%)

## Claims

1. A method for preparing a hardened calcium sulfate dihydrate block comprising mixing calcium sulfate hemihydrate powder, preferably alpha-type calcium sulfate hemihydrate powder, with an aqueous solution containing phosphate ions so as to form a paste, wherein the aqueous solution has a pH value lower than 10, and forming a block of hardened calcium sulfate dihydrate from said paste, wherein said aqueous solution is an aqueous solution of (NH₄)₃PO₄, (NH₄)₂HPO₄, NH₄H₂PO₄, K₃PO₄, K₂HPO₄, Na₃PO₄, NaH₂PO₄, H₃PO₄, or a mixture thereof.

2. The method of claim 1, wherein there is no alkaline compound being added to the aqueous solution, the calcium sulfate hemihydrate power or the paste thereof to adjust pH values thereof.

3. The method of claim 1, wherein the aqueous solution or the calcium sulfate hemihydrate powder has a temperature lower than 50°C before said mixing, and said mixing is conducted at a temperature lower than 50°C.

4. The method of claim 3, wherein the aqueous solution has a concentration of the phosphate ions lower than 1.0 M, preferably is of 0.01 M to 0.5 M.

5. The method of claim 1, wherein the mixing is conducted with a liquid to powder ratio of 0.20 cc/g to 0.60 cc/g, preferably 0.30 cc/g to 0.50 cc/g.

6. The method of claim 1, wherein said aqueous solution is an aqueous solution of (NH₄)₃PO₄, (NH₄)₂HPO₄, NH₄H₂PO₄, or a mixture thereof.

7. The method of claim 1, further comprising introducing the paste to a hole or cavity and letting the paste become set in-situ to form a block of hardened calcium sulfate dihydrate in the hole or cavity.

8. The method of claim 1, further comprising shaping the paste in a mold, and removing the mold to form a block of hardened calcium sulfate dihydrate.

9. The method of claim 8, further comprising pressurizing said paste in said mold before said paste becomes set to remove a portion of liquid from said paste, so that a liquid to powder ratio of said paste decreases, the pressure applied to the paste in the mold is form about 1 MPa to 500 MPa, preferably from 100 MPa to 500 MPa.

10. The method of claim 1, further comprising mixing a pore-forming agent with the powder or with the paste; shaping the paste in a mold; removing the mold to form a block of hardened calcium sulfate dihydrate with the pore-forming agent embedded therein; and immersing said block of hardened calcium sulfate dihydrate with the pore-forming agent embedded therein in an immersing liquid to dissolve said pore-forming agent in the immersing liquid, creating pores therein, so that a porous block having a porosity of 50-90 vol% is formed.

11. The method of claim 10, wherein the pore forming agent is selected from the group consisting of LiCl, KCl, NaCl, MgCl₂, CaCl₂, NaIO₃, KI, Na₃PO₄, K₃PO₄, Na₂CO₃, amino acid-sodium salt, amino acid-potassium salt, glucose, polysaccharide, fatty acid-sodium salt, fatty acid-potassium salt, potassium bitartrate (KHC₄H₄O₆), potassium carbonate, potassium gluconate (KC₆H₁₁O₇), potassium-sodium tartrate (KNaC₄H₄O₆·4H₂O), potassium sulfate (K₂SO₄), sodium sulfate, sodium lactate and mannitol.

12. The method of claim 10, wherein the immersing liquid is water or a phosphate-containing solution having a phosphate concentration from 0.1 M to 6 M, preferably from 1 M to 3 M.

13. The method of claim 8 or 10, further comprising impregnating the block or the porous block with an impregnating liquid for a period of time, so that a compressive strength of the resulting impregnated block or the resulting impregnated porous block removed from the impregnating liquid is increased compared to that of said block or said porous block without said impregnating treatment.

14. The method of claim 13, wherein the impregnating liquid is a phosphate-containing solution having a phosphate concentration from 0.1 M to 6 M, preferably from 1 M to 3 M.

15. The method of claim 8 or 10, further comprising breaking up the block or the porous block into pellets.

## Patentansprüche

1. Verfahren zur Herstellung eines gehärteten Calciumsulfat-Dihydrat-Blocks, umfassend das Mischen von Calciumsulfat-Halbhydrat-Pulver, vorzugsweise Calciumsulfat-Halbhydrat-Pulver vom Alpha-Typ, mit einer wässrigen Lösung, die Phosphationen enthält, um eine Paste zu bilden, wobei die wässrige Lösung einen pH-Wert von weniger als 10 aufweist, und Bilden eines Blocks aus gehärtetem Calciumsulfat-Dihydrat aus der Paste, wobei die wässrige Lösung eine wässrige Lösung von (NH₄)₃PO₄, (NH₄)₂HPO₄, NH₄H₂PO₄, K₃PO₄, K₂HPO₄, Na₃PO₄, NaH₂PO₄, H₃PO₄ oder eine Mischung davon ist.

2. Das Verfahren nach Anspruch 1, wobei der wässrigen Lösung, dem Calciumsulfat-Halbhydrat-Pulver oder der Paste davon keine alkalische Verbindung zugesetzt wird, um deren pH-Werte einzustellen.

3. Das Verfahren nach Anspruch 1, wobei die wässrige Lösung oder das Calciumsulfat-Halbhydrat-Pulver vor dem Mischen eine Temperatur von weniger als 50°C hat und das Mischen bei einer Temperatur von weniger als 50°C durchgeführt wird.

4. Das Verfahren nach Anspruch 3, wobei die wässrige Lösung eine Konzentration an Phosphationen von weniger als 1,0 M, vorzugsweise von 0,01 M bis 0,5 M aufweist.

5. Das Verfahren nach Anspruch 1, wobei das Mischen mit einem Flüssigkeits-zu-Pulver-Verhältnis von 0,20 cc/g bis 0,60 cc/g, vorzugsweise 0,30 cc/g bis 0,50 cc/g, durchgeführt wird.

6. Das Verfahren nach Anspruch 1, wobei die wässrige Lösung eine wässrige Lösung von (NH₄)₃PO₄, (NH₄)₂HPO₄, NH₄H₂PO₄ oder eine Mischung davon ist.

7. Das Verfahren nach Anspruch 1, ferner umfassend das Einbringen der Paste in ein Loch oder einen Hohlraum und das In-situ-Abbindenlassen der Paste, um einen Block aus gehärtetem Calciumsulfat-Dihydrat in dem Loch oder Hohlraum zu bilden.

8. Das Verfahren nach Anspruch 1, ferner umfassend das Formen der Paste in einer Form und das Entfernen der Form, um einen Block aus gehärtetem Calciumsulfat-Dihydrat zu bilden.

9. Das Verfahren nach Anspruch 8, ferner umfassend das Unter-Druck-Setzen der Paste in der Form, bevor die Paste fest wird, um einen Teil der Flüssigkeit aus der Paste zu entfernen, so dass ein Flüssigkeits-Pulver-Verhältnis der Paste abnimmt, wobei der auf die Paste in der Form ausgeübte Druck etwa 1 MPa bis 500 MPa, vorzugsweise von 100 MPa bis 500 MPa, beträgt.

10. Das Verfahren nach Anspruch 1, ferner umfassend das Mischen eines porenbildenden Mittels mit dem Pulver oder mit der Paste; das Formen der Paste in einer Form; das Entfernen der Form, um einen Block aus gehärtetem Calciumsulfat-Dihydrat mit dem darin eingebetteten porenbildenden Mittel zu bilden; und das Eintauchen des Blocks aus gehärtetem Calciumsulfat-Dihydrat mit dem darin eingebetteten porenbildenden Mittel in eine Eintauchflüssigkeit, um das porenbildende Mittel in der Eintauchflüssigkeit aufzulösen und darin Poren zu erzeugen, so dass ein poröser Block mit einer Porosität von 50-90 Vol.-% gebildet wird.

11. Das Verfahren nach Anspruch 10, wobei das porenbildende Mittel ausgewählt ist aus der Gruppe, bestehend aus LiCI, KCl, NaCl, MgCl₂, CaCl₂, NaIO₃, KI, Na₃PO₄, K₃PO₄, Na₂CO₃, Aminosäure-Natriumsalz, Aminosäure-Kaliumsalz, Glucose, Polysaccharid, Fettsäure-Natriumsalz, Fettsäure-Kaliumsalz, Kaliumbitartrat (KHC₄H₄O₆), Kaliumcarbonat, Kaliumgluconat (KC₆H₁₁O₇), Kalium-Natriumtartrat (KNaC₄H₄O₆·4H₂O), Kaliumsulfat (K₂SO₄), Natriumsulfat, Natriumlactat und Mannitol.

12. Das Verfahren nach Anspruch 10, wobei die Eintauchflüssigkeit Wasser oder eine phosphathaltige Lösung mit einer Phosphatkonzentration von 0,1 M bis 6 M, vorzugsweise von 1 M bis 3 M, ist.

13. Das Verfahren nach Anspruch 8 oder 10, ferner umfassend das Imprägnieren des Blocks oder des porösen Blocks mit einer Imprägnierflüssigkeit für eine Zeitspanne, so dass eine Druckfestigkeit des resultierenden imprägnierten Blocks oder des resultierenden imprägnierten porösen Blocks, der aus der Imprägnierflüssigkeit entfernt wurde, im Vergleich zu der des Blocks oder des porösen Blocks ohne die Imprägnierbehandlung erhöht wird.

14. Das Verfahren nach Anspruch 13, wobei die Imprägnierflüssigkeit eine phosphathaltige Lösung mit einer Phosphatkonzentration von 0,1 M bis 6 M, vorzugsweise von 1 M bis 3 M, ist.

15. Das Verfahren nach Anspruch 8 oder 10, ferner umfassend das Zerkleinern des Blocks oder des porösen Blocks in Pellets.

## Revendications

1. Procédé de préparation d'un bloc de sulfate de calcium durci dihydraté comprenant le mélange d'une poudre de sulfate de calcium hémihydraté, préférablement d'une poudre de sulfate de calcium de type alpha hémihydraté, avec une solution aqueuse contenant des ions phosphates afin de former une pâte, la solution aqueuse ayant une valeur de pH inférieure à 10, et formant un bloc de sulfate de calcium durci dihydraté à partir de ladite pâte, ladite solution aqueuse étant une solution aqueuse de (NH₄)₃PO₄, (NH₄)₂HPO₄, NH₄H₂PO₄, K₃PO₄, K₂HPO₄, Na₃PO₄, NaH₂PO₄, H₃PO₄, ou un mélange de ceux-ci.

2. Procédé selon la revendication 1, dans lequel aucun composé alcalin n'est ajouté à la solution aqueuse, la poudre de sulfate de calcium hémihydraté ou la pâte de celui-ci pour ajuster ses valeurs de pH.

3. Procédé selon la revendication 1, la solution aqueuse ou la poudre de sulfate de calcium hémihydraté ayant une température inférieure à 50°C avant ledit mélange, et ledit mélange étant conduit à une température inférieure à 50°C.

4. Procédé selon la revendication 3, la solution aqueuse ayant une concentration des ions phosphates inférieure à 1,0 M, préférablement étant de 0,01 M à 0,5 M.

5. Procédé selon la revendication 1, le mélange étant conduit avec un rapport liquide sur poudre de 0,20 cc/g à 0,60 cc/g, préférablement de 0,30 cc/g à 0,50 cc/g.

6. Procédé selon la revendication 1, ladite solution aqueuse étant une solution aqueuse de (NH₄)₃PO₄, (NH₄)₂HPO₄, NH₄H₂PO₄, ou un mélange de ceux-ci.

7. Procédé selon la revendication 1, comprenant en outre l'introduction de la pâte dans un trou ou une cavité et le fait de laisser la pâte durcir *in situ* pour former un bloc de sulfate de calcium durci dihydraté dans le trou ou la cavité.

8. Procédé selon la revendication 1, comprenant en outre la mise en forme de la pâte dans un moule, et le retrait du moule pour former un bloc de sulfate de calcium durci dihydraté.

9. Procédé selon la revendication 8, comprenant en outre la mise sous pression de ladite pâte dans ledit moule avant que ladite pâte durcisse pour retirer une portion du liquide de ladite pâte, de sorte qu'un rapport liquide sur poudre de ladite pâte baisse, la pression appliquée à la pâte dans le moule étant d'environ 1 MPa à 500 MPa, préférablement de 100 MPa à 500 MPa.

10. Procédé selon la revendication 1, comprenant en outre le mélange d'un agent formant pore avec la poudre ou avec la pâte ; la mise en forme de la pâte dans un moule ; le retrait du moule pour former un bloc du sulfate de calcium durci dihydraté avec l'agent formant pore enchâssé à l'intérieur ; et l'immersion dudit bloc de sulfate de calcium durci dihydraté avec l'agent formant pore enchâssé à l'intérieur dans un liquide d'immersion pour dissoudre ledit agent formant pore dans le liquide d'immersion, créant des pores à l'intérieur, de sorte qu'un bloc poreux ayant une porosité de 50 à 90 % en vol est formé.

11. Procédé selon la revendication 10, l'agent formant pore étant sélectionné dans le groupe constitué de LiCl, KCl, NaCl, MgCl₂, CaCl₂, NaIO₃, KI, Na₃PO₄, K₃PO₄, Na₂CO₃, du sel sodique d'acide aminé, du sel potassique d'acide aminé, du glucose, d'un polysaccharide, d'un sel sodique d'acide gras, d'un sel potassique d'acide gras, du bitartrate de potassium (KHC₄H₄O₆), du carbonate de potassium, du gluconate de potassium (KC₆H₁₁O₇), du tartrate de potassium-sodium (KNaC₄H₄O₆·4H₂O), du sulfate de potassium (K₂SO₄), du sulfate de sodium, du lactate de sodium et du mannitol.

12. Procédé selon la revendication 10, le liquide d'immersion étant l'eau ou une solution contenant du phosphate ayant une concentration en phosphate de 0,1 M à 6 M, préférablement de 1 M à 3 M.

13. Procédé selon la revendication 8 ou 10, comprenant en outre l'imprégnation du bloc ou du bloc poreux avec un liquide d'imprégnation sur une période de temps, de sorte qu'une force de compression du bloc imprégné résultant ou du bloc poreux imprégné résultant retiré du liquide d'imprégnation est accrue comparée à celle dudit bloc ou dudit bloc poreux sans ledit traitement d'imprégnation.

14. Procédé selon la revendication 13, le liquide d'imprégnation étant une solution contenant du phosphate ayant une concentration en phosphate de 0,1 M à 6 M, préférablement de 1 M à 3 M.

15. Procédé selon la revendication 8 ou 10, comprenant en outre le fait de briser le bloc ou le bloc poreux en pastilles.
